# EUROPEAN PATENT SPECIFICATION

(11) **EP 3 801 320 B1**
(45) Date of publication and mention of the grant of the patent: **18.03.2026**
(21) Application number: 19810332.7
(22) Date of filing: 27.03.2019
(51) Int. Cl.: A61B 17/72, A61B 17/80, A61B 17/86, A61B 17/68

(54) **BONE FIXATION IMPLANT**
KNOCHENFIXIERIMPLANTAT
IMPLANT DE FIXATION OSSEUSE

(30) Priority: 31.05.2018 US 201862678530 P
(43) Date of publication of application: 14.04.2021
(73) Proprietor: Wright Medical Technology, Inc., Memphis, TN 38117 (US)
(72) Inventor: MULLER, Erin, Lakeland, TN 38002 (US); THOREN, Brian, Robert, Memphis, TN 38122 (US)
(74) Representative: Zimmermann & Partner Patentanwälte mbB
(86) International application number: PCT/US2019/024227
(87) International publication number: WO 2019/231531

(56) References cited:
- WO-A1-2011/031416
- WO-A1-2013/075730
- US-A1- 2006 100 624
- US-A1- 2007 191 855
- US-A1- 2010 057 214
- US-A1- 2013 204 250
- US-A1- 2016 354 127
- US-A1- 2017 196 602

## Description

### FIELD

This disclosure relates generally to medical devices, and more specifically to implants for correcting bone deformity.

### BACKGROUND

Hallux valgus deformities in the human foot relate to a condition in which the first (great) toe has a deviated position leaning in towards the second toe. The first metatarsal deviates towards the mid-sagittal plane, and the great toe deviates away from the mid-sagittal plane. This is often accompanied by a bump due to a swollen bursal sac or a bony anomaly on the metatarsophalangeal joint.

A variety of non-surgical methods are used to treat hallux valgus, but in cases of continued pain or visible deformity, the patient may seek a surgical correction of the condition. Surgical methods may include removing the bony enlargement of the first metatarsal, realigning the first metatarsal bone relative to the adjacent metatarsal bone, and/or straightening the great toe relative to the first metatarsal and adjacent toes.

One such method of treating hallux valgus deformities is known as a Lapidus procedure. In a Lapidus procedure the first tarsal-metatarsal joint is fused to decrease the movement of the joint. This straightens the first metatarsal and toe to reduce or eliminate the hallux valgus deformity.

Document US 2007/0191855 A1 relates to a fracture fixation device and implantation jig therefor.

Document US 2016/0354127 A1 relates to a combined intramedullary-extramedullary bone stabilization and alignment system.

Document US 2013/0204250 A1 relates to a method and apparatus for treating a bone fracture.

### SUMMARY

The invention is defined in appended claim 1.

In one embodiment, an implant is configured to attach a first bone to a second bone. The implant includes an intramedullary portion, an extramedullary portion, and an intermediate portion. The intramedullary portion is configured for insertion into the first bone and has a longitudinal axis and an intramedullary fastener hole that extends through the intramedullary portion. The extramedullary portion is configured for contact with the second bone and has a bone facing surface and an extramedullary fastener hole extending through the extramedullary portion. The bone facing surface is configured to abut a surface of the second bone and is spaced apart from the longitudinal axis. The intermediate portion extends between the intramedullary portion and the extramedullary portion and has a compression fastener hole having a compression fastener hole axis extending therethrough. The compression fastener hole axis is disposed at an oblique angle with respect to the longitudinal axis.

In another aspect, an implant system is configured to attach a first bone to a second bone. The implant system includes a first fastener, a second fastener, a compression screw, and an implant. The implant includes an intramedullary portion, an extramedullary portion, and an intermediate portion. The intramedullary portion is configured for insertion into the first bone and has a longitudinal axis and an intramedullary fastener hole that extends through the intramedullary portion. The extramedullary portion is configured for contact with the second bone and has a bone facing surface and an extramedullary fastener hole extending through the extramedullary portion. The bone facing surface is configured to abut a surface of the second bone and is spaced apart from the longitudinal axis. The intermediate portion extends between the intramedullary portion and the extramedullary portion and has a compression fastener hole having a compression fastener hole axis extending therethrough. The compression fastener hole axis is disposed at an acute angle with respect to the longitudinal axis.

Further disclosed but not claimed is a method of securing a metatarsal bone to a tarsal bone The method includes creating an incision to access a tarsal-metatarsal joint. The method further includes forming a longitudinal hole in the metatarsal bone. The method further includes inserting an intramedullary portion of an implant into the longitudinal hole. The intramedullary portion has a longitudinal axis and an intramedullary fastener hole. The implant also has an extramedullary portion having an extramedullary fastener hole. The extramedullary portion has a bone facing surface spaced apart from the longitudinal axis. The implant also has an intermediate portion extending between the intramedullary portion and the extramedullary portion. The intramedullary portion has a compression fastener hole. The method further includes forming a first drill hole in the metatarsal bone. The method further includes inserting a first fastener into the first drill hole and the intramedullary fastener hole to attach the intramedullary portion to the metatarsal bone. The method further includes forming a compression drill hole through the metatarsal bone and into the tarsal bone. The method further includes inserting a compression screw into the compression fastener hole and the compression drill hole. The method further includes reducing a distance between the metatarsal bone and the tarsal bone. The method further includes forming a second drill hole in the tarsal bone. The method further includes inserting a second fastener through the extramedullary fastener hole and the second drill hole to attach the extramedullary portion to the tarsal bone.

### BRIEF DESCRIPTION OF THE DRAWINGS

These and other features and advantages of the bone fixation implants and methods of implantation described herein will be more fully disclosed in, or rendered obvious by, the following detailed description of the preferred embodiments, which is to be considered together with the accompanying drawings wherein like numbers refer to like parts and further wherein:
FIG. 1 shows a top view of an implant, according to one embodiment.
FIG. 2 shows a side view of the implant of FIG. 1.
FIG. 3 shows a side cross-sectional view of the implant of FIG. 1.
FIG. 4 shows a top view of an target guide, according to one embodiment.
FIG. 5 shows a side view of the target guide of FIG. 4.
FIG. 6 shows a side cross-sectional view of the target guide of FIG. 4.
FIG. 7 shows a top view of the target guide of FIG. 4 engaged with the implant of FIG. 1.
FIG. 8 shows a side view of the target guide of FIG. 4 engaged with the implant of FIG. 1.
FIG. 9 shows a side cross-sectional view of the target guide of FIG. 4 engaged with the implant of FIG. 1.
FIG. 10 shows a side view of a drill guide configured for use with the target guide of FIG. 4.
FIG. 11 shows a cross-sectional view of the drill guide of FIG. 10.
FIG. 12 shows a side view of the implant of FIG. 1 implanted in a metatarsal bone.
FIG. 13 shows a perspective view of the implant of FIG. 1 implanted in a metatarsal bone.
FIG. 14 shows another perspective view of the implant of FIG. 1 implanted in a metatarsal bone.
FIG. 15 is a flow chart of a method of treatment using the implant of FIG. 1 and the target guide of FIG. 4.

### DETAILED DESCRIPTION

This description of preferred embodiments is intended to be read in connection with the accompanying drawings, which are to be considered part of the entire written description of this invention. The drawing figures are not necessarily to scale and certain features of the invention may be shown exaggerated in scale or in somewhat schematic form in the interest of clarity and conciseness. In the description, relative terms such as "horizontal," "vertical," "up," "down," "top," and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

This disclosure provides an implant and a target guide for surgical fixation of the first tarsal-metatarsal joint as well as methods for implantation and securing of the implant. The disclosed methods are not claimed. The implant is suitable for correction of hallux valgus deformity of the first metatarsal and can also be used in the correction of analogous deformities in other joints. Although the drawings show application of the implant and target guide to treat a first metatarsal for correction of hallux valgus, the implant and target guide can be sized and configured to treat other bones, and can also be used in a variety of procedures.

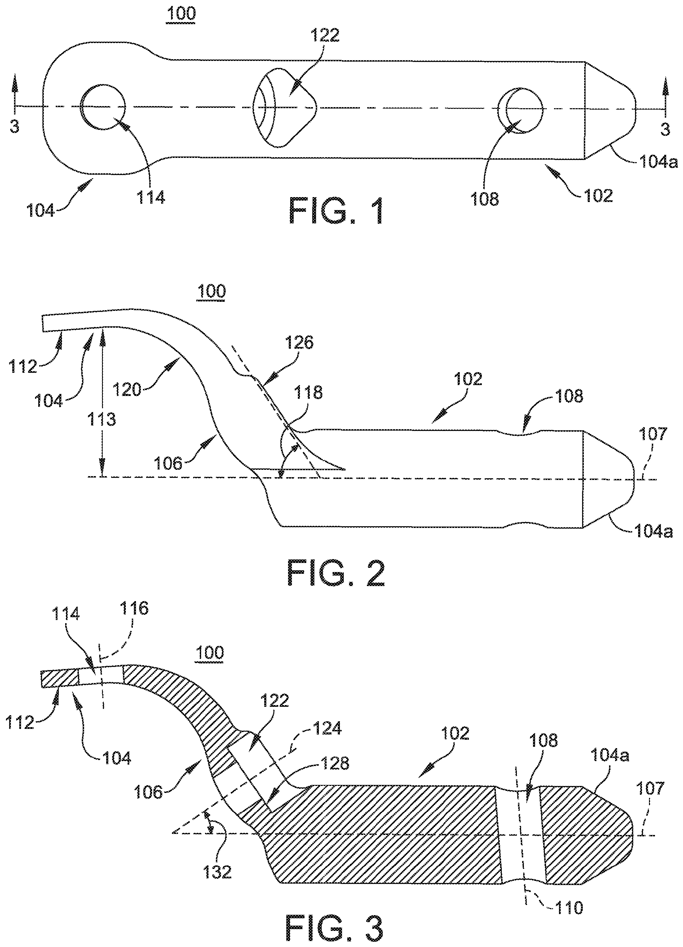

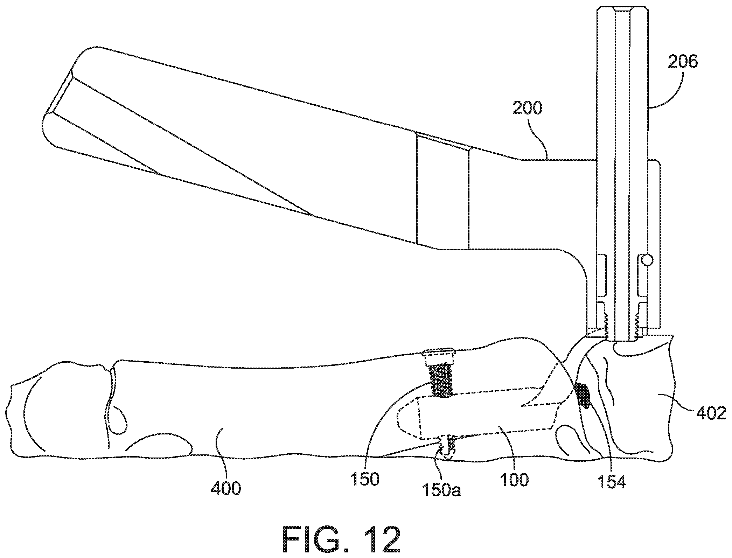

In one embodiment, as shown in FIGS. 1-3, an implant 100 includes an intramedullary portion 102, an extramedullary portion 104, and an intermediate portion 106. In the embodiment shown, the intramedullary portion 102, the extramedullary portion 104, and the intermediate portion 106 are integrally formed from a monolithic component. In another embodiment, one or more of the intramedullary portion 102, the extramedullary portion 104, and the intermediate portion 106 are separate components that are joined using fasteners, adhesive, welding, or any other appropriate technique. The implant 100 is configured to join a first bone 400 to a second bone 402, as shown in FIGS. 12-14 and as described further herein. It should be noted that the implant 100 can be used on either the left or right foot.

The intramedullary portion 102 is configured for insertion into a first bone 400, such as the first metatarsal. The intramedullary portion 102 has a longitudinal axis 107, which can be a central axis. In one embodiment, the intramedullary portion 102 includes a cylindrical portion. The distal end of the intramedullary portion 102 can include a chamfer 104a to assist with insertion into the first bone. In alternative embodiments, the entire intramedullary portion 102 is tapered (*i.e.,* the intramedullary portion forms a portion of a cone). Alternatively, the distal end of the intramedullary portion 102 can taper to an edge (*i.e.,* forming a triangular prism). The taper may be bi-lateral (*i.e.,* tapering from both the top and bottom of the intramedullary portion 102) or unilateral (*i.e.,* tapering from only the top or bottom of the intramedullary portion 102). In other embodiments, the distal end of the intramedullary portion 102 is pyramidally shaped.

Although the cross-sectional geometry of intramedullary portion 102 is shown as being cylindrical, in other embodiments the cross-sectional geometry of intramedullary portion 102 may be polygonal (*e.g.,* triangular, rectangular, pentagonal, etc.) and/or include one or more protrusions or flat surfaces formed thereon to resist rotation of the implant 100 relative to the first bone segment or fragment. In some embodiments, the intramedullary portion 102 may be completely or partially threaded. In some embodiments, the intramedullary portion 102 may include one or more fins or protrusions extending outwardly therefrom to resist rotation of the implant 100 relative to the bone segment, section, or fragment.

An intramedullary fastener hole 108 extends through the intramedullary portion 102. In various embodiments, the intramedullary fastener hole 108 may be cylindrical or slotted. The intramedullary fastener hole 108 has an intramedullary fastener hole axis 110, shown in FIG. 3. In one embodiment, the intramedullary fastener hole axis 110 is substantially orthogonal to the longitudinal axis 107 and is oriented such that the intramedullary fastener hole axis 110 extends in a substantially superior-inferior orientation when the implant 100 is implanted. In another embodiment, the intramedullary fastener hole axis 110 forms an oblique angle with the longitudinal axis 107. For example, the intramedullary fastener hole axis 110 can be oriented in a superior-proximal to inferior-distal orientation with respect to the longitudinal axis 107. In one embodiment, the intramedullary fastener hole 108 is threaded. By providing a threaded intramedullary fastener hole 108, movement of a fastener engaged with the intramedullary fastener hole 108 relative to the implant 100 is minimized. This can reduce or eliminate fretting of the fastener and/or implant. In another embodiment, the intramedullary fastener hole 108 is unthreaded. Providing a press or slip fit between the fastener and the intramedullary fastener hole 108 can also minimize relative movement. In at least one embodiment, the intramedullary portion 102 includes multiple fastener holes.

The extramedullary portion 104 is configured for contact with a second bone 402 (FIGS. 12-14), such as a tarsal bone. The extramedullary portion 104 includes a bone facing surface 112, shown in FIGS. 2 and 3, offset from the longitudinal axis 107. The bone facing surface 112 is configured to abut a surface of the second bone when the implant 100 is implanted, as shown in FIGS. 12-14. In one embodiment, the bone facing surface 112 is offset from the intramedullary portion 102 such that when the intramedullary portion 102 is inserted into the first metatarsal the bone facing surface 112 sits atop the tarsal bone. The bone facing surface 112 is spaced from the longitudinal axis 107 a distance 113 that is greater than one half the width of the intramedullary portion 102 (*i.e.,* greater than the radius of the intramedullary portion 102). The distance 113 can be configured to provide the desired offset based on the severity of the deformity. In some embodiments, a kit containing multiple implants is provided, the implants having a variety of offset distances 113. For example, the implants can have an offset distance 113 of 2mm, 4mm, 6mm, 8mm, or any other appropriate value. The increment of offset distances between implants can be any appropriate or desired increment. The bone facing surface 112 can be in contact with the tarsal bone or, alternatively, a gap can be present between the bone facing surface 112 and the tarsal bone. In one embodiment, the bone facing surface 112 is substantially flat. In another embodiment, the bone facing surface 112 is contoured to conform to the tarsal bone. For example, the bone facing surface 112 can be at least partially concave.

An extramedullary fastener hole 114 extends through the extramedullary portion 104. The extramedullary fastener hole 114 has an extramedullary fastener hole axis 116. In one embodiment, the extramedullary fastener hole axis 116 extends in a substantially superior-inferior orientation. The intramedullary fastener hole axis 110 and the extramedullary fastener hole axis 116 are substantially parallel. In at least one embodiment, the extramedullary portion 104 includes multiple fastener holes.

The intermediate portion 106 extends between the intramedullary portion 102 and the extramedullary portion 104. As shown in FIG. 2, the intermediate portion 106 is inclined relative to the longitudinal axis 107 such that the superior surface 126 of the intermediate portion 106 forms an angle 118 with the longitudinal axis 107. In one embodiment, the angle 118 is about 60°. In another embodiment, the angle 118 is between about 55° and about 65°. In another embodiment, the angle 118 is between about 45° and about 75°. As described above with respect to the distance 113 between the longitudinal axis 107 and the extramedullary portion 104, the angle 118 can be selected based on the severity of the deformity. As the distance 113 is increased to accommodate more severe deformities, the angle 118 may be increased as well. A kit having a plurality of implants can be provided having a variety of angles. In one embodiment, the implant 100 includes a fillet 120 at the intersection of the extramedullary portion 104 and the intermediate portion 106 to provide a smooth contour for contact with the tarsal bone.

A compression fastener hole 122 extends through the intermediate portion 106. The compression fastener hole 122 has a compression fastener hole axis 124. In one embodiment, the compression fastener hole axis 124 is orthogonal to the superior surface 126 of the intermediate portion 106. In another embodiment, the compression fastener hole axis 124 forms a non-perpendicular angle with the superior surface 126. The compression fastener hole 122 is configured to receive a compression screw therein such that the shaft of the compression screw extends into the tarsal bone. The compression fastener hole axis 124 forms an oblique angle 132 with the longitudinal axis 107. For example, in one embodiment, the angle 132 is about 35°. In another embodiment, the angle 132 is between about 30° and about 40°. In another embodiment, the angle 132 is between about 25° and about 45°. In one embodiment, the compression fastener hole axis 124 also forms an oblique angle with respect to the intramedullary fastener hole axis 110 and the extramedullary screw hole axis.

The compression fastener hole 122 includes a shoulder 128 extending into the hole such that a counterbore is formed therein. In one embodiment, as will be described further below, as the compression screw is installed in the tarsal bone, the head of the compression screw contacts the shoulder 128, thereby pulling the metatarsal and tarsal bones toward one another.

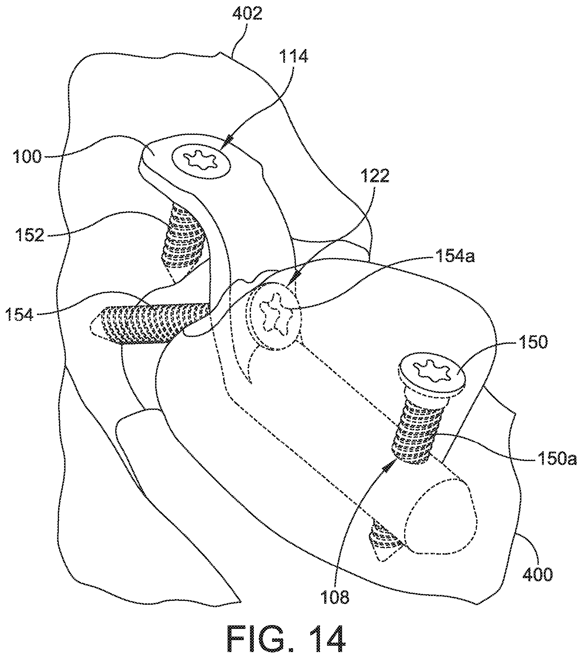

As shown in FIG. 14, the intramedullary fastener hole 108 is configured to receive a fastener 150 that extends through a portion of the first metatarsal. The fastener 150 can be a screw, pin, nail, k-wire, rod, or any other appropriate fastener. As shown, in one embodiment, the fastener 150 is a screw and the shaft 150a of the screw extends through the intramedullary fastener hole 108. The fastener 150 fixes the implant 100 to the metatarsal to restrict movement of the implant 100 relative to the metatarsal. As mentioned above, the intramedullary fastener hole 108 can be threaded to engage a threaded shaft 150a of the fastener 150. Alternatively, the fastener 150 can be self-tapping such that it forms threads in the intramedullary fastener hole 108 as it is inserted.

Further, the extramedullary fastener hole 114 is configured to receive a fastener 152 that extends into the tarsal bone. This secures the implant 100 to the tarsal bone. In one embodiment, the extramedullary fastener hole 114 allows for variable angle alignment. In some embodiments, polyaxial screws such as 3Di locking screws or non-locking screws sold by Wright Medical Technology, Inc. of Memphis, TN may be utilized. For example, in one embodiment, the fastener 152 is a locking screw and the shaft of the screw can form an angle of up to about 15° in any direction with respect to the extramedullary fastener hole axis 116.

The compression fastener hole 122 is configured to receive a compression fastener 154, as shown in FIG. 14. The compression fastener 154 is configured to pass through the compression fastener hole 122 and engage the tarsal bone. In one embodiment, the compression fastener 154 is a screw. In one embodiment, the head 154a of the compression fastener 154 contacts the shoulder 128 as the compression fastener 154 is inserted. As the compression fastener 154 is tightened, it draws the metatarsal bone towards the tarsal bone. In other words, the space between the metatarsal and tarsal bones is reduced, compressing the bones together. The amount of compression can be controlled by the surgeon by controlling the amount that the compression fastener 154 is turned within the compression fastener hole 122 (and, thereby, the metatarsal bone).

In some embodiments, the compression fastener 154 is an interfragmentary fastener. In such embodiments, the threaded portion of the compression fastener 154 may engage both the metatarsal and tarsal bones. In other embodiments, the threaded portion of the compression fastener 154 engages only the tarsal bone.

In one embodiment, as described in more detail below, the compression fastener 154 is installed prior to the fastener 152 in extramedullary portion 104. As a result, when the fastener 152 is inserted into the tarsal bone, the relative positions of the metatarsal and tarsal bones are fixed.

The implant 100 can comprise a metal, such as titanium, stainless steel, or CoCr. In some embodiments, the implant 100 can comprise a metal substrate coated with or having an additional layer of hydroxyapatite (HA), titanium plasma spray (TPS) / vacuum plasma spray (VPS), roughened surface of resorbable blast media (RBM), a bioactive glass, an antimicrobial or antibiotic, or strontium. Alternatively, the implant 100 can comprise a metal substrate with a composite coating or composite layer including HA on plasma, beads, an irregular sintered coating or TPS on an RBM-prepared substrate. In other embodiments, the metal substrate can have a porous coating, such as spherical bead, asymmetrical powder or an irregular particle coating.

In some embodiments, the metal substrate of implant 100 comprises a degradable (resorbable) material, such as a magnesium alloy, which may contain lithium, aluminum, rare earth metals (e.g., neodymium or cerium), manganese, zinc or other metals. In other embodiments, the resorbable material can include, but is not limited to polymer materials including a polylactide, polyglycolide, polycaprolactone, polyvalerolactone, polycarbonates, polyhydroxy butyrates, poly ortho esters, polyurethanes, polyanhydrides, and combinations and copolymers thereof, for example.

In some embodiments, the implant 100 comprises a biologic material. The biologic material can be a combination of Medical grade β-TCP granules and rhPDGF-BB solution, such as AUGMENT^{®} bone graft material sold by Wright Medical Technology, Inc. of Memphis, TN. The biologic material can be applied, sprayed, or inserted at the wound site for bone in-growth, or can be provided as a coating on the implants or any or all portions of the implant system. In some embodiments, the biologic material is a coating containing osteoinductive or osteoconductive biological components. In some embodiments, the biologic material can include bone morphogenetic factors, i.e., growth factors whose activity are specific to bone tissue including, but not limited to, demineralized bone matrix (DBM), bone protein (BP), bone morphogenetic protein (BMP), and mixtures and combinations thereof. Additionally, formulations for promoting the attachment of endogenous bone may comprise bone marrow aspirate, bone marrow concentrate, and mixtures and combinations thereof.

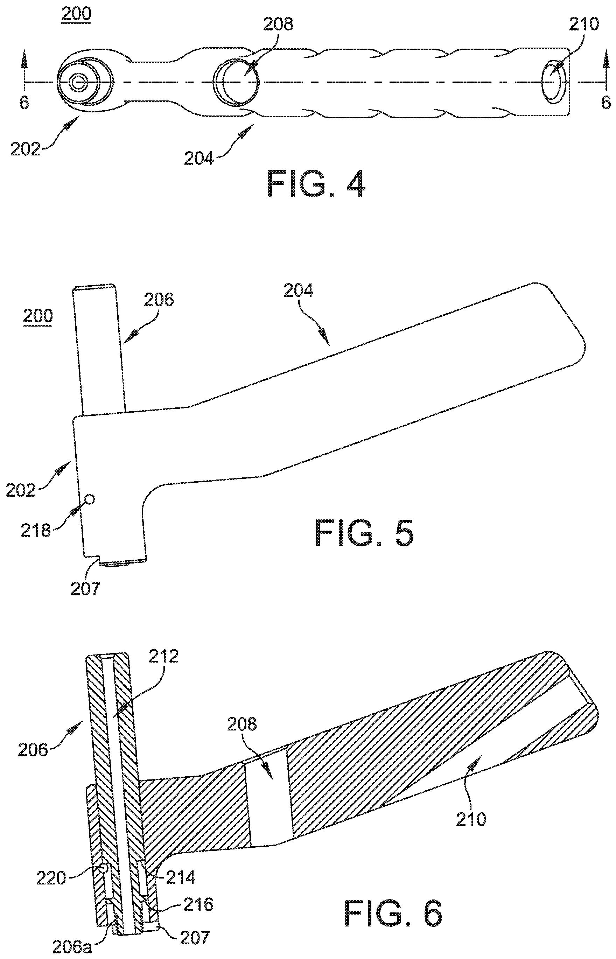

FIGS. 4-6 show a target guide 200 that includes a coupling portion 202 and an arm 204. The target guide 200 can be integrally constructed from a monolithic component. Alternatively, the target guide 200 can be constructed of two or more separate components that are joined using fasteners, adhesive, or any other appropriate means. The target guide 200 is suitable for guiding drills to form fastener holes in a bone and for insertion of the intramedullary portion 102 into the first bone 400. The target guide 200 can also be used to rotate the implant 100 and metatarsal to achieve the desired alignment of the metatarsal and tarsal bone, as will be described in more detail herein. The use of a target guide to both guide a drill as well as provide the desired rotation simplifies the correction of the deformity and eliminates the need for additional fixtures or tools.

The coupling portion 202 is configured to couple to the extramedullary portion 104. In one embodiment, the coupling portion 202 includes an insert 206 having a threaded end 206a for coupling to the extramedullary fastener hole 114. The coupling portion 202 also includes a flange 207 extending therefrom and configured to contact the lateral and medial sides of the extramedullary portion 104 to align the implant 100 to the target guide 200. The implant 100 can also be aligned to the target guide 200 through any other appropriate means, such as through the use of one or more pins. The target guide 200 is coupled to the implant 100 prior to implantation and is used to guide insertion of the implant 100 into the metatarsal.

In one embodiment, the insert 206 includes a first flange 214 and a second flange 216. Further, the coupling portion 202 includes a pin hole 218. After inserting the insert 206 in the coupling portion 202, a pin 220 is inserted in the pin hole 218 to retain the insert 206 within the coupling portion 202, as shown in FIG. 6. The distance between the first flange 214 and the second flange 216 allows the threaded end 206a to be engaged and disengaged from the extramedullary fastener hole 114. In addition, the insert 206 is freely rotatable within the coupling portion 202 to allow for rotational engagement with the extramedullary fastener hole 114. The pin 220 can be press-fit within the pin hole 218.

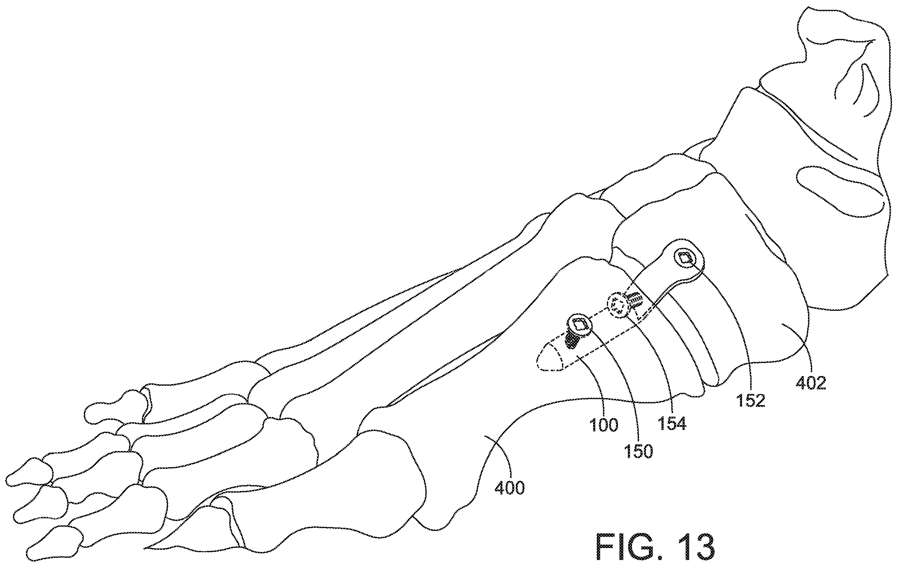

The target guide described above is only exemplary and is not limiting. For example, in a variation of the target guide (not shown), the insert 206 is not pre-assembled within the coupling portion 202, and the pin 220 is omitted. The surgeon or technician can assemble the insert 206 (or a drill guide having the same outer diameter as the insert 206) inside the coupling portion 202 before use. With a removable insert 206 or drill guide, the surgeon can remove the insert 206 or drill guide and implant the fastener 152 (FIGS. 13 and 14) through the coupling portion 202 of the target guide 200, without first removing the target guide 200. This provides greater flexibility in surgical technique and procedures.

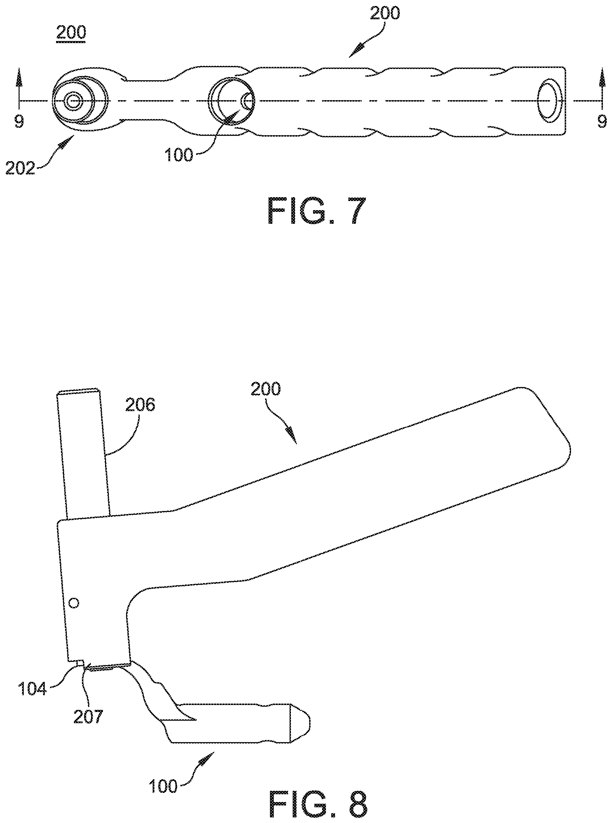

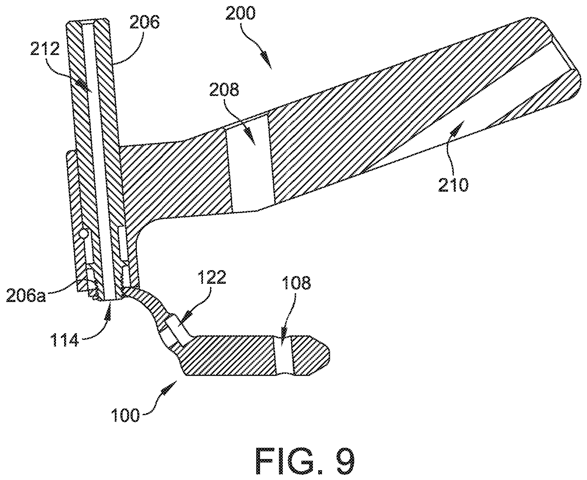

FIGS. 7-9 show the target guide 200 coupled to the implant 100. The arm 204 includes an intramedullary guide aperture 208 and a compression guide aperture 210. As shown in FIG. 9, when the target guide 200 is connected to the implant 100, the intramedullary guide aperture 208 is configured to be aligned with the intramedullary fastener hole 108 of the implant 100. The compression guide aperture 210 is configured to align with the compression fastener hole 122 of the implant 100. As will be described further below, the intramedullary 208 and compression 210 guide apertures can guide a drill bit as it forms holes through the metatarsal or tarsal bones.

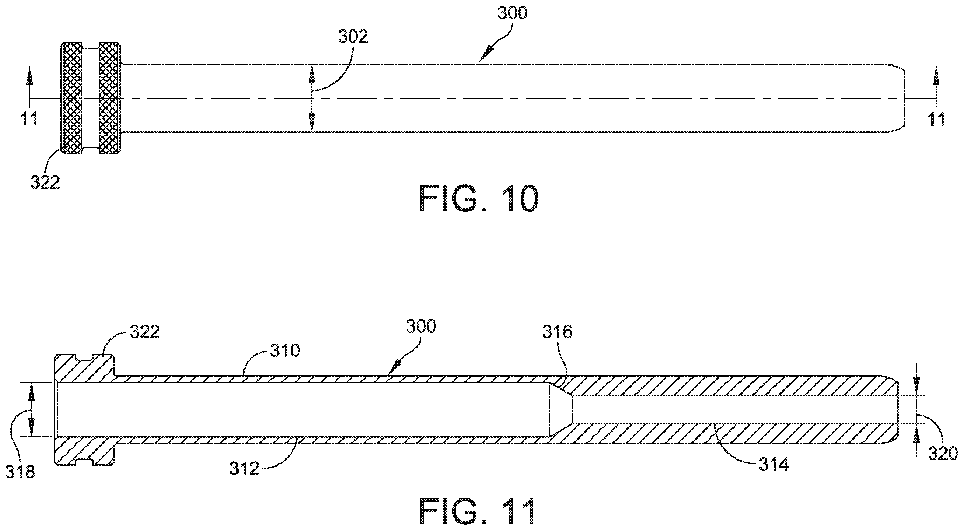

FIGS. 10 and 11 show an example of a drill guide 300 suitable for use with the target guide 200. FIG. 10 is a plan view of the drill guide 300, and FIG. 11 is a cross-section of the drill guide 300, taken along section line 11-11 of FIG. 10.

In FIGS. 10 and 11, the drill guide 300 has an outer surface 310 with an outer diameter 302 sized to be slidably received in the intramedullary guide aperture 208 and/or the compression guide aperture 210 of target guide 200. The drill guide 300 has a first portion with a bore 312 having a first inner diameter 318. The drill guide 300 has a second portion with a bore 314 having a second inner diameter 320 less than the first inner diameter 318. The second inner diameter 320 is sized to slidably receive and align a drill that penetrates the drill guide 300 and the first 400 and/or second 402 bones. The first inner diameter 318 of the bore 312 of drill guide 300 is sized larger than the second inner diameter 320, to avoid friction between the drill and the sidewall of bore 312. The drill guide 300 has a taper section 316 between (and connecting) the bore 312 and the bore 314, for guiding the drill 350 into the bore 314. The drill guide 300 may also have a knob 322 with a larger diameter than the outer surface 310. The knob 322 acts as a stop to prevent the drill guide 300 from falling through the arm 204, The knob 322 can have a gripping surface, such as ridges, groov es, splines, or a knurled, patterned or textured surface.

In some methods, the surgeon inserts a longitudinal k-wire (not shown) in the metatarsal 400 and uses a cannulated reamer (not shown) to form a longitudinal intramedullary opening in the metatarsal 400 concentric with the longitudinal k-wire. The surgeon removes the longitudinal k-wire from the longitudinal intramedullary opening and inserts the intramedullary portion 102 of the implant 100 into the longitudinal intramedullary opening,

In some methods, the surgeon applies a force to the target guide 200 or a k-wire or drill inserted in the target guide 200, resulting in application of a moment to rotate the implant 100 and the metatarsal about the longitudinal axis 107 of the intramedullary portion 102 of implant 100. Although the surgeon can apply the force directly to the target guide 200, in some instances the surgeon may wish to grasp a drill or k-wire and use the drill or k-wire as a joy stick during the rotation. The surgeon applies the force to rotate the implant 100 until the metatarsal rotates through a desired angle. After rotation, the extramedullary portion 104 of the implant 100 and the tarsal bone 402 are properly aligned with respect to the metatarsal 400,

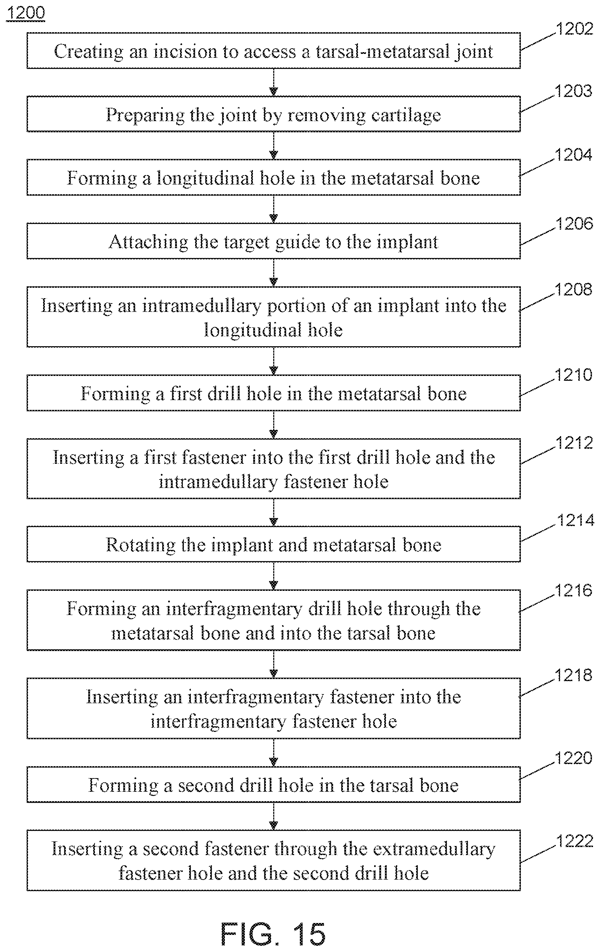

Further disclosed but not claimed and, shown in FIG. 15, is a method 1200 of fixing a first bone to a second bone. At step 1202, the surgeon creates an incision that provides access to the tarsal-metatarsal joint. The incision can be formed with any appropriate surgical tool, such as a scalpel. Next, at step 1203, the joint is prepared by removing cartilage, tissue, and/or other material in order to provide access to the first and second bones. Various tools can be used to prepare the joint.

At step 1204, the surgeon forms the longitudinal hole in the metatarsal bone (for receiving the intramedullary portion of the implant). Optionally, prior to forming the longitudinal hole, a k-wire can be inserted into the metatarsal to define the orientation of the longitudinal hole. A cannulated reamer, guided by the k-wire, can be used to form the longitudinal hole. After forming the longitudinal hole, the k-wire is removed.

At step 1206, the surgeon attaches the target guide to the extramedullary fastener hole in the extramedullary portion of the implant (by engaging the threaded end of the insert of the target guide with the threads of the extramedullary fastener hole). Alternatively, the surgeon can obtain a pre-packaged or previously assembled construct comprising an implant attached to the threaded end of the insert of a target guide.

At step 1208, the surgeon inserts the intramedullary portion of the implant into the longitudinal intramedullary opening in the proximal section of the first metatarsal. During the insertion, the surgeon may grip the target guide to push the implant into the opening. When the insertion is completed, the extramedullary portion of the implant has a bone facing surface facing radially inward toward the first longitudinal axis.

At step 1210, the surgeon forms a first drill hole in the first metatarsal. In some methods the surgeon inserts a k-wire through the body of the target guide prior to forming the first drill hole in order to guide the drill as it forms the first fastener hole.

At step 1212, a first fastener is inserted into the first drill hole and through the intramedullary portion of the implant to secure the implant to the metatarsal. In one method, the first fastener is a screw.

At step 1214, after inserting the first fastener, the surgeon applies a force to the target guide to rotate the implant and the first metatarsal about the first longitudinal axis in situ to correct a hallux valgus deformity. Because the implant is fixed to the metatarsal by the first fastener, rotation of the implant results in a corresponding rotation of the metatarsal. Hence, no additional tools or fixtures are required to impart the desired rotation.

In other methods, the implant and metatarsal are rotated prior to insertion of the first fastener. For example, after forming the first drill hole, the surgeon may maintain the drill in the first drill hole and use the drill like a joy stick to manipulate and rotate the implant and the first metatarsal to achieve the desired rotation angle.

At step 1216, the surgeon forms a compression drill hole through the metatarsal and tarsal bone using the target guide and a drill guide. Optionally, prior to forming the compression drill hole, a k-wire is inserted to guide the orientation of the compression drill hole. In such a method, the drill may be cannulated to allow the drill to pass over the k-wire. After forming the compression drill hole, the k-wire can be removed.

At step 1218, after forming the compression drill hole, the surgeon inserts a fastener through the compression fastener hole in the implant and into the compression drill hole in the tarsal bone. In some methods, the compression screw has a cannula, and the inserting step comprises inserting the compression screw in the compression fastener hole with the k-wire extending through the cannula of the compression screw.

At step 1220, the surgeon forms a drill hole in the tarsal bone. The drill hole can be formed using a drill guided by the insert of the target guide. Alternatively, a dedicated drill guide can be used to guide the drill. In some methods, a k-wire is inserted through the extramedullary fastener hole and into the tarsal bone prior to forming the drill hole in order to guide the drill.

At step 1222, a fastener is inserted through the extramedullary fastener hole and into the drill hole. In some methods, the target guide is removed from the implant prior to insertion of the second fastener.

Optionally, the method may further include making a second incision to provide clearance for insertion of the compression screw and making a third incision to provide clearance for insertion of the first fastener,

## Claims

1. An implant (100) configured to attach a first bone (400) to a second bone (402), the implant (100) comprising:
an intramedullary portion (102) configured for insertion into the first bone (400), the intramedullary portion (102) having:
a longitudinal axis (107), and
an intramedullary fastener hole (108) extending through the intramedullary portion (102);
an extramedullary portion (104) configured for contact with the second bone (402), the extramedullary portion (104) having:
a bone facing surface (112) configured to abut a surface of the second bone (402), the bone facing surface (112) spaced apart from the longitudinal axis (107), and
an extramedullary fastener hole (114) extending through the extramedullary portion (104); and
an intermediate portion (106) extending between the intramedullary portion (102) and the extramedullary portion (104), the intermediate portion (106) having a compression fastener hole (122) having a compression fastener hole axis (124) extending therethrough;
wherein the compression fastener hole axis (124) is disposed at an oblique angle with respect to the longitudinal axis (107), **characterized in that** the intramedullary fastener hole (108) has an intramedullary fastener hole axis (110) and the extramedullary fastener hole (114) has an extramedullary fastener hole axis (116) and the intramedullary fastener hole axis (110) and the extramedullary fastener hole axis (116) are parallel.

2. The implant (100) of claim 1, wherein a shoulder extends into the compression fastener hole (122), the shoulder configured to contact a head of a screw inserted in the compression fastener hole (122).

3. The implant (100) of claim 1, wherein the first bone (400) is a metatarsal bone and the second bone (402) is a tarsal bone.

4. The implant (100) of claim 1, wherein the intramedullary fastener hole (108) is configured to receive a fastener to fix the intramedullary portion (102) to the first bone (400) and the extramedullary fastener hole (114) is configured to receive a fastener to fix the extramedullary portion (104) to the second bone (402).

5. The implant (100) of claim 4, wherein a shoulder extends into the compression fastener hole (122) and the compression fastener hole (122) is configured to receive a fastener having a head and a threaded shaft such that the head contacts the shoulder and the threaded shaft is configured to extend into and engage the second bone (402).

6. The implant (100) of claim 1, wherein the extramedullary fastener hole (114) is threaded.

7. The implant (100) of claim 1, wherein the longitudinal axis (107) is a central axis and the intramedullary portion (102) has a width, and wherein a distance (113) from the longitudinal axis (107) to the bone facing surface (112) of the extramedullary portion (104) is greater than one half of the width.

8. An implant system configured to attach a first bone (400) to a second bone (402), the implant system comprising:
a first fastener, a second fastener, and a compression screw; and
the implant (100) of any of the preceding claims.

9. The implant system of claim 8, wherein the first fastener is configured to be inserted through the intramedullary fastener hole (108) to secure the intramedullary portion (102) to the first bone (400), the second fastener is configured to be inserted through the extramedullary fastener hole (114) and secure the extramedullary portion (104) to the second bone (402), and the compression screw is configured to be inserted through the compression fastener hole (122) and engage the second bone (402).

10. The implant system of claim 8, wherein a shoulder extends into the compression fastener hole (122), the shoulder configured to contact a head of the compression screw when it is inserted in the compression fastener hole (122).

11. The implant system of claim 8, wherein the compression screw is an interfragmentary screw.

12. The implant system of claim 8, wherein the longitudinal axis (107) is a central axis and the intramedullary portion (102) has a width, and wherein a distance from the longitudinal axis (107) to the bone facing surface (112) of the extramedullary portion (104) is greater than one half the width.

13. The implant system of claim 8, further comprising a target guide comprising:
a coupling portion adapted to engage the extramedullary fastener hole (114) of the implant (100);
an arm having a first guide aperture and a compression guide aperture;
wherein when the coupling portion is engaged with the extramedullary fastener hole (114) the first guide aperture is aligned with the intramedullary fastener hole (108) of the implant and the compression guide aperture is aligned with the compression fastener hole.

14. The implant system of claim 13, further comprising a flange extending from the coupling portion and configured to contact a lateral side of the implant (100).

## Patentansprüche

1. Implantat (100), das zum Anbringen eines ersten Knochens (400) an einen zweiten Knochen (402) konfiguriert ist, wobei das Implantat (100) umfasst:
einen intramedullären Abschnitt (102), der zur Einführung in den ersten Knochen (400) konfiguriert ist, wobei der intramedulläre Abschnitt (102) aufweist:
eine Längsachse (107), und
ein intramedulläres Befestigungselementloch (108), das sich durch den intramedullären Abschnitt (102) erstreckt;
einen extramedullären Abschnitt (104), der für den Kontakt mit dem zweiten Knochen (402) konfiguriert ist, wobei der extramedulläre Abschnitt (104) aufweist:
eine knochenzugewandte Oberfläche (112), die zum Anliegen an einer Oberfläche des zweiten Knochens (402) konfiguriert ist, wobei die knochenzugewandte Oberfläche (112) von der Längsachse (107) beabstandet ist, und
ein extramedulläres Befestigungselementloch (114), das sich durch den extramedullären Abschnitt (104) erstreckt; und
einen Zwischenabschnitt (106), der sich zwischen dem intramedullären Abschnitt (102) und dem extramedulären Abschnitt (104) erstreckt, wobei der Zwischenabschnitt (106) ein Kompressionsbefestigungselementloch (122) mit einer Kompressionsbefestigungselementlochachse (124) aufweist, die sich durch dieses hindurch erstreckt;
wobei die Kompressionsbefestigungselementlochachse (124) in einem schrägen Winkel bezüglich der Längsachse (107) angeordnet ist,
**dadurch gekennzeichnet, dass** das intramedulläre Befestigungselementloch (108) eine intramedulläre Befestigungselementlochachse (110) aufweist und das extramedulläre Befestigungselementloch (114) eine extramedulläre Befestigungselementlochachse (116) aufweist und die intramedulläre Befestigungselementlochachse (110) und die extramedulläre Befestigungselementlochachse (116) parallel sind.

2. Implantat (100) nach Anspruch 1, wobei sich eine Schulter in das Kompressionsbefestigungselementloch (122) erstreckt, wobei die Schulter zum Kontaktieren eines Kopfes einer in das Kompressionsbefestigungselementloch (122) eingesetzten Schraube konfiguriert ist.

3. Implantat (100) nach Anspruch 1, wobei der erste Knochen (400) ein Mittelfußknochen und der zweite Knochen (402) ein Fußwurzelknochen ist.

4. Implantat (100) nach Anspruch 1, wobei das intramedulläre Befestigungselementloch (108) zum Aufnehmen eines Befestigungselements konfiguriert ist, um den intramedullären Abschnitt (102) an dem ersten Knochen (400) zu befestigen, und das extramedulläre Befestigungselementloch (114) zum Aufnehmen eines Befestigungselements konfiguriert ist, um den extramedullären Abschnitt (104) an dem zweiten Knochen (402) zu befestigen.

5. Implantat (100) nach Anspruch 4, wobei sich eine Schulter in das Kompressionsbefestigungselementloch (122) erstreckt und das Kompressionsbefestigungselementloch (122) zum Aufnehmen eines Befestigungselements mit einem Kopf und einem Gewindeschaft konfiguriert ist, so dass der Kopf die Schulter kontaktiert und der Gewindeschaft dazu konfiguriert ist, sich in den zweiten Knochen (402) zu erstrecken und mit diesem in Eingriff zu stehen.

6. Implantat (100) nach Anspruch 1, wobei das extramedulläre Befestigungselementloch (114) mit einem Gewinde versehen ist.

7. Implantat (100) nach Anspruch 1, wobei die Längsachse (107) eine Mittelachse ist und der intramedulläre Abschnitt (102) eine Breite aufweist, und wobei ein Abstand (113) von der Längsachse (107) zu der knochenzugewandten Oberfläche (112) des extramedullären Abschnitts (104) größer als die Hälfte der Breite ist.

8. Implantatsystem, das zum Befestigen eines ersten Knochens (400) an einem zweiten Knochen (402) konfiguriert ist, wobei das Implantatsystem umfasst:
ein erstes Befestigungselement, ein zweites Befestigungselement und eine Kompressionsschraube; und
das Implantat (100) nach einem der vorstehenden Ansprüche.

9. Implantatsystem nach Anspruch 8, wobei das erste Befestigungselement zum Einführen durch das intramedulläre Befestigungselementloch (108) konfiguriert ist, um den intramedullären Abschnitt (102) an dem ersten Knochen (400) zu befestigen, das zweite Befestigungselement zum Einführen durch das extramedulläre Befestigungselementloch (114) und zum Befestigen des extramedullären Abschnitts (104) an dem zweiten Knochen (402) konfiguriert ist, und die Kompressionsschraube zum Einführen durch das Kompressionsbefestigungselementloch (122) und zum Eingriff mit dem zweiten Knochen (402) konfiguriert ist.

10. Implantatsystem nach Anspruch 8, wobei sich eine Schulter in das Kompressionsbefestigungselementloch (122) erstreckt, wobei die Schulter zum Kontaktieren eines Kopfes der Kompressionsschraube konfiguriert ist, wenn diese in das Kompressionsbefestigungselementloch (122) eingesetzt wird.

11. Implantatsystem nach Anspruch 8, wobei die Kompressionsschraube eine interfragmentäre Schraube ist.

12. Implantatsystem nach Anspruch 8, wobei die Längsachse (107) eine Mittelachse ist und der intramedulläre Abschnitt (102) eine Breite aufweist, und wobei ein Abstand von der Längsachse (107) zu der knochenzugewandten Oberfläche (112) des extramedullären Abschnitts (104) größer als die Hälfte der Breite ist.

13. Implantatsystem nach Anspruch 8, ferner umfassend eine Zielführung, umfassend:
einen Kopplungsabschnitt, der dazu angepasst ist, mit dem extramedulären Befestigungselementloch (114) des Implantats (100) in Eingriff zu treten;
einen Arm mit einer ersten Führungsöffnung und einer Kompressionsführungsöffnung;
wobei, wenn der Kopplungsabschnitt mit dem extramedulären Befestigungselementloch (114) in Eingriff steht, die erste Führungsöffnung mit dem intramedulären Befestigungselementloch (108) des Implantats ausgerichtet ist und die Kompressionsführungsöffnung mit dem Kompressionsbefestigungselementloch ausgerichtet ist.

14. Implantatsystem nach Anspruch 13, ferner umfassend einen Flansch, der sich von dem Kopplungsabschnitt erstreckt und zum Kontaktieren einer lateralen Seite des Implantats (100) konfiguriert ist.

## Revendications

1. Implant (100) configuré pour fixer un premier os (400) à un deuxième os (402), l'implant (100) comprenant :
une partie intramédullaire (102) configurée pour être insérée dans le premier os (400), la partie intramédullaire (102) présentant :
un axe longitudinal (107), et
un trou de fixation intramédullaire (108) s'étendant à travers la partie intramédullaire (102) ;
une partie extramédullaire (104) configurée pour entrer en contact avec le deuxième os (402), la partie extramédullaire (104) présentant :
une surface faisant face à l'os (112) configurée pour venir en butée contre une surface du deuxième os (402), la surface faisant face à l'os (112) étant espacée de l'axe longitudinal (107), et
un trou de fixation extramédullaire (114) s'étendant à travers la partie extramédullaire (104) ; et
une partie intermédiaire (106) s'étendant entre la partie intramédullaire (102) et la partie extramédullaire (104), la partie intermédiaire (106) présentant un trou de fixation par compression (122) présentant un axe de trou de fixation par compression (124) s'étendant à travers lui ;
dans lequel l'axe de trou de fixation par compression (124) est disposé selon un angle oblique par rapport à l'axe longitudinal (107), **caractérisé en ce que** le trou de fixation intramédullaire (108) présente un axe de trou de fixation intramédullaire (110) et le trou de fixation extramédullaire (114) présente un axe de trou de fixation extramédullaire (116) et l'axe de trou de fixation intramédullaire (110) et l'axe de trou de fixation extramédullaire (116) sont parallèles.

2. Implant (100) selon la revendication 1, dans lequel un épaulement s'étend dans le trou de fixation par compression (122), l'épaulement étant configuré pour entrer en contact avec une tête d'une vis insérée dans le trou de fixation par compression (122).

3. Implant (100) selon la revendication 1, dans lequel le premier os (400) est un os métatarsien et le deuxième os (402) est un os tarsien

4. Implant (100) selon la revendication 1, dans lequel le trou de fixation intramédullaire (108) est configuré pour recevoir une fixation pour fixer la partie intramédullaire (102) au premier os (400) et le trou de fixation extramédullaire (114) est configuré pour recevoir une fixation pour fixer la partie extramédullaire (104) au deuxième os (402).

5. Implant (100) selon la revendication 4, dans lequel un épaulement s'étend dans le trou de fixation par compression (122) et le trou de fixation par compression (122) est configuré pour recevoir une fixation présentant une tête et un arbre fileté de telle sorte que la tête entre en contact avec l'épaulement et l'arbre fileté est configuré pour s'étendre dans le deuxième os (402) et se mettre en prise avec lui.

6. Implant (100) selon la revendication 1, dans lequel le trou de fixation extramédullaire (114) est fileté.

7. Implant (100) selon la revendication 1, dans lequel l'axe longitudinal (107) est un axe central et la partie intramédullaire (102) présente une largeur, et dans lequel une distance (113) de l'axe longitudinal (107) à la surface faisant face à l'os (112) de la partie extramédullaire (104) est supérieure à une moitié de la largeur.

8. Système d'implant configuré pour fixer un premier os (400) à un deuxième os (402), le système d'implant comprenant :
une première fixation, une deuxième fixation et une vis à compression ; et
l'implant (100) selon l'une quelconque des revendications précédentes.

9. Système d'implant selon la revendication 8, dans lequel la première fixation est configurée pour être insérée à travers le trou de fixation intramédullaire (108) pour ancrer la partie intramédullaire (102) au premier os (400), la deuxième fixation est configurée pour être insérée à travers le trou de fixation extramédullaire (114) et ancrer la partie extramédullaire (104) au deuxième os (402), et la vis à compression est configurée pour être insérée à travers le trou de fixation par compression (122) et mettre en prise le deuxième os (402).

10. Système d'implant selon la revendication 8, dans lequel un épaulement s'étend dans le trou de fixation par compression (122), l'épaulement étant configuré pour entrer en contact avec une tête de la vis à compression lorsqu'il est inséré dans le trou de fixation par compression (122).

11. Système d'implant selon la revendication 8, dans lequel la vis à compression est une vis interfragmentaire.

12. Système d'implant selon la revendication 8, dans lequel l'axe longitudinal (107) est un axe central et la partie intramédullaire (102) présente une largeur, et dans lequel une distance de l'axe longitudinal (107) à la surface faisant face à l'os (112) de la partie extramédullaire (104) est supérieure à une moitié de la largeur.

13. Système d'implant selon la revendication 8, comprenant en outre un guide cible comprenant :
une partie de couplage adaptée pour mettre en prise le trou de fixation extramédullaire (114) de l'implant (100) ;
un bras présentant une première ouverture de guidage et une ouverture de guidage par compression ;
dans lequel lorsque la partie de couplage est en prise avec le trou de fixation extramédulaire (114), la première ouverture de guidage est alignée avec le trou de fixation intramédullaire (108) de l'implant et l'ouverture de guidage par compression est alignée avec le trou de fixation par compression.

14. Système d'implant selon la revendication 13, comprenant en outre un rebord s'étendant à partir de la partie de couplage et configuré pour entrer en contact avec un côté latéral de l'implant (100).
